(19) Europäisches Patentamt
European Patent Office
Office européen des brevets

(11) Publication number: 0 015 544 B1

(12) **EUROPEAN PATENT SPECIFICATION**

(45) Date of publication of patent specification: 05.01.83

(21) Application number: 80101108.1

(22) Date of filing: 05.03.80

(51) Int. Cl.³: C 07 C 149/23, A 61 K 31/195, A 61 K 31/22 //C07C103/46

(54) **Derivatives of the mercaptopropionamido-acetic acid, process for the preparation thereof and compositions.**

(30) Priority: 06.03.79 IT 2076979
06.03.79 IT 2077079

(43) Date of publication of application:
17.09.80 Bulletin 80/19

(45) Publication of the grant of the patent:
05.01.83 Bulletin 83/1

(84) Designated Contracting States:
AT BE CH DE FR GB IT LU NL SE

(56) References cited:
EP - A - 0 001 989
DE - A - 1 967 088
DE - A - 2 810 261

The file contains technical information submitted after the application was filed and not included in this specification

(73) Proprietor: ZAMBON S.p.A.
Via Cappuccini, 40
I-36100 Vicenza (IT)

(72) Inventor: Signor, Angelo
Via Monte Gallo, 21
I-35100 Padova (IT)
Inventor: Cima, Lorenzo
Via Beato Pellegrino, 96
I-35100 Padova (IT)

(74) Representative: Marchi, Massimo et al,
c/o Zambon S.p.A. Patent & Licensing Dept. Via Lillo del Duca, 10
I-20091 Bresso (Milano) (IT)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 015 544 B1

## Derivatives of the mercaptopropionamido-acetic acid, process for the preparation thereof and compositions

The present invention relates to a new class of derivatives of the alpha or beta-mercaptopropionamido-acetic acid, to the process for the preparation thereof and to the use of said derivatives as active ingredient in pharmaceutical and cosmetic compositions. More particularly, the present invention relates to the compounds comprised in the general formula

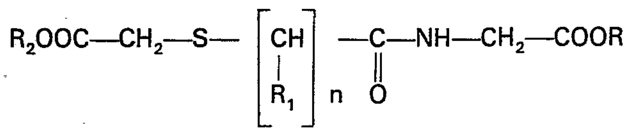

(I)

wherein R is H or 2—20 C alkyl radical; $R_1$ is H or $CH_3$; $n$ is 1 or 2 with the provision that when $n = 1$; $R_1$ is $CH_3$ and when $n = 2$, $R_1$ is H; $R_2$ is H or $\frac{1}{2}$Ca.

The derivatives of the alpha-mercaptopropionamido-acetic acid of the present invention are represented by the general formula

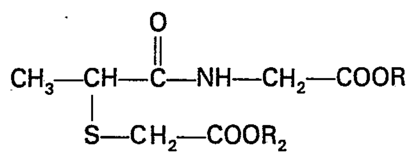

(II)

The derivatives of the beta-mercaptopropionamido-acetic acid are represented by the general formula

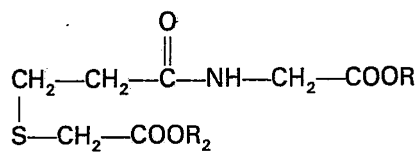

(III)

Both in formula (II) and (III) R and $R_2$ have the above mentioned meanings.

According to the present invention the compounds of the formula (I) are prepared by reacting the glycine or an ester thereof with a halide of the alpha-bromo or beta-bromo-propionic acid.

The thus obtained derivative of the bromo-propionamido-acetic acid is then directly reacted with the thioglycolic acid.

The new process is illustrated by the following reaction scheme:

a) 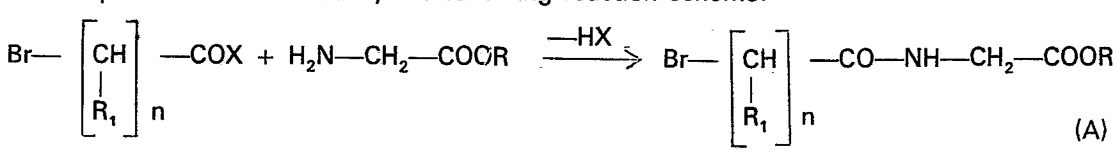


(A)

(wherein R, $R_1$ and $n$ have the above meanings; X = Cl, Br, I)

b) 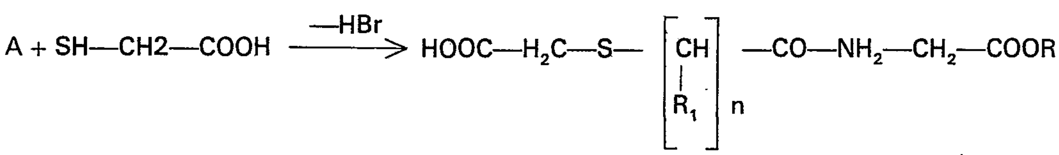


wherein R, $R_1$ and $n$ have the above defined meanings.

The step (a) of the above schematized process is carried out in an inert organic solvent, not miscible with $H_2O$, such as for instance methylene chloride, chloroform, ethyl acetate, and in the presence of an organic base, in at least stoichiometric amount on the starting materials, able to block the hydrohalogenic acid as soon as it is formed.

The reaction is carried out under strong stirring by cooling in water-ice bath, in order to preferably keep a temperature not overcoming 10°C.

The compound (A) thus prepared is separated by concentrating the solution up to dryness and by crystallizing the residue from a suitable organic solvent. The bromo-propionamido-acetic acid or the ester thereof, obtained in step (a) are allowed to react with the thioglycolic acid in an aprotic, dipolar organic solvent, miscible with water, such as for instance DMFA, DMSO, in the presence of at least a stoichiometric amount of an organic base able to block the HBr which is formed.

The reaction is carried out under stirring, at room temperature.

In the event that the free acid is allowed to react directly, the reaction can be preferably carried out in water.

The final product (I) which is formed in very high yields is separated, after acidification, by different techniques according to the particular product thus formed; in the most favourable cases a filtration is sufficient; in some cases an extraction with organic solvent is necessary.

In order to make easier the performance of the process according to the present invention, some examples for the preparation of the new products of this invention are given hereinafter. These examples are only illustrative and do not limit in any way the present invention.

Example 1

To a suspension of 15.4 g (0.05 mol) of glycine myristyl ester hydrochloride (prepared by exchange reaction between glycine methyl ester and myristyl alcohol) in 150 ml of methylene chloride, kept under strong stirring and cooled in a ice-water bath, there are added 12.1 g (0.12 mol) of triethylamine and dropwise 8.6 g (0.055 mol) of alpha-bromopropionyl chloride. After completion of the addition the reaction mixture is kept under stirring for further 3 hrs; the solution is then washed with 5% sodium bicarbonate, water and then dried on anhydrous sodium sulfate. After filtration the solution is concentrated to dryness under vacuum and the residual solid product is recrystallized from methanol to give 16.5 g of alpha-bromopropionyl-glycine myristyl ester (yield 81% of the theoric). The elemental analysis has given the following results for $C_{19}H_{36}NO_3Br$:

| | C | H | N | Br |
|---|---|---|---|---|
| calculated % | 56.15 | 8.92 | 3.44 | 19.66 |
| found % | 56.23 | 8.85 | 3.40 | 19.50 |

m.p. 68—69°C.

10.5 g (0.105 mol) of triethylamine and 4.15 g (0.045 mol) of thioglycolic acid are added to a solution of 12.2 g (0.03 mol) of alpha-bromopropionyl-glycine myristyl ester in 100 ml of anhydrous dimethyl formamide. The reaction mixture is kept for 4 hrs under stirring at room temperature; then it is poured into 500 ml of cold water and acidified at pH $\simeq$2 with concentrated HCl the formed solid is filtered off, washed with water and crystallized from ethanol-water.

11.3 g of alpha-(S-carboxymethyl)-propionylglycine myristyl ester are obtained (yield 90% on the theoric). The elemental analysis has given the following results for $C_{21}H_{39}NSO_5$:

| | C | H | N | S |
|---|---|---|---|---|
| calculated % | 60.40 | 9.41 | 3.35 | 7.67 |
| found % | 60.04 | 9.69 | 3.30 | 7.31 |

m.p. 54—55°C.

Analogously, the following compounds have been prepared:

— lauryl ester of the alpha-(S-carboxymethyl)-propionylglycine
— cetyl ester of the alpha-(S-carboxymethyl)-propionylglycine
— myristyl ester of the beta-(S-carboxymethyl)-propionylglycine
— lauryl ester of the beta-(S-carboxymethyl)-propionylglycine
— cetyl ester of the beta-(S-carboxymethyl)-propionylglycine.

The characterizing features of the intermediates as well as of the final products are summarized in the following tables I and II.

Example 2

To a solution of 10.5 g (0.05 mol) of beta-bromopropionyl-glycine (obtained as described in the foregoing example and dissolved in 100 ml of water) kept under stirring and room temperature, 15 g of potassium carbonate and 4.6 g (0.05 mol) of thioglycolic acid are added.

The reaction is continued for 2 hrs, then the solution is acidified at pH 2 with 2.5 M sulfuric acid and is continuously extracted overnight with ethyl acetate. The organic solvent is removed under vacuum and the obtained solid residue is crystallized from acetone. 8.1 g of beta-(S-carboxymethyl)-propionyl-glycine are obtained (yield 73% on the theoric).

The elemental analysis has given the following results for $C_7H_{11}NSO_5$:

| | C | H | N | S |
|---|---|---|---|---|
| calculated % | 38.0 | 5.01 | 6.33 | 14.49 |
| found % | 37.64 | 5.16 | 6.23 | 14.19 |

m.p. 129—130°C.

TABLE I

*Bromopropionylglycine esters*
*alpha and beta bromopropionylglycine cetyl ester — M.W. 434.46*

| calculated for $C_{21}H_{40}NO_3Br$ | C | H | N | Br | m.p. |
|---|---|---|---|---|---|
| | 58.05 | 9.28 | 3.22 | 18.39 | |
| found % alpha | 58.39 | 9.45 | 3.11 | 18.21 | 73° |
| beta | 58.72 | 9.68 | 3.17 | 17.00 | 80° |

*alpha e beta bromopropionylglycine myristyl ester — M.W. 406.4*

| calculated for $C_{19}H_{36}NO_3Br$ | C | H | N | Br | m.p. |
|---|---|---|---|---|---|
| | 56.15 | 8.92 | 3.44 | 19.66 | |
| found % alpha | 56.23 | 8.85 | 3.40 | 19.50 | 68—69° |
| beta | 55.92 | 8.80 | 3.38 | 19.42 | 74—75° |

*alpha e beta bromopropionylglycine lauryl ester — M.W. 378.35*

| calculated for $C_{17}H_{32}NO_3Br$ | C | H | N | Br | m.p. |
|---|---|---|---|---|---|
| | 53.96 | 8.51 | 3.70 | 21.12 | |
| found % alpha | 53.98 | 8.63 | 3.67 | 21.06 | 63—64° |
| beta | 54.44 | 8.56 | 3.69 | 20.41 | 70—71° |

TABLE II

*S-carboxymethyl-propionyl-glycine — M.W. 221.2*

| calculated for $C_7H_{11}NSO_5$ | C | H | N | S | m.p. |
|---|---|---|---|---|---|
| | 38.00 | 5.01 | 6.33 | 14.49 | |
| found % alpha | — | — | — | — | oil |
| beta | 37.64 | 5.16 | 6.23 | 14.19 | 129—130° |

*S-carboxymethyl-propionyl-glycine lauryl ester — M.W. 389.6*

| calculated for $C_{19}H_{35}NSO_5$ | C | H | N | S | m.p. |
|---|---|---|---|---|---|
| | 58.58 | 9.05 | 3.59 | 8.23 | |
| found % alpha | 58.11 | 8.90 | 3.40 | 8.61 | 59—60° |
| beta | 58.88 | 9.02 | 3.56 | 8.10 | 98—102° |

*S-carboxymethyl-propionyl-glycine myristyl ester — M.W. 417.6*

| calculated for $C_{21}H_{39}NSO_5$ | C | H | N | S | m.p. |
|---|---|---|---|---|---|
| | 60.40 | 9.41 | 3.35 | 7.67 | |
| found % alpha | 60.04 | 9.69 | 3.30 | 7.31 | 54—55° |
| beta | 60.73 | 9.27 | 3.26 | 7.85 | 98—101° |

*S-carboxymethyl-propionyl-glycine cetyl ester — M.W. 445.7*

| calculated for $C_{23}H_{43}NSO_5$ | C | H | N | S | m.p. |
|---|---|---|---|---|---|
| | 61.98 | 9.72 | 3.14 | 7.19 | |
| found % alpha | 61.70 | 9.64 | 3.12 | 7.00 | |
| beta | 61.84 | 9.51 | 3.07 | 7.36 | 104—107° |

It is known that the lowering of the sputum viscosity by chemical means is a fundamental therapeutical aim in the modern therapy of the obstructive chronic bronchopneumopathies.

The bronchial secretion usually consists of purulent exudates and mucus, the former rich in DNA, the latter in mucoproteins; the chemical compounds used thereto as fluidifying agents are enzymes, surfactants and sulfurated compounds.

However, the two first groups of products are endowed with not constant activity and poor tolerability (allergies, attacks of bronchial asthma, irritation of mucosae), so that the sulfurated compounds, in particular the L-cysteine and some derivatives thereof are more favourable as mucolytics; the derivatives of the L-cysteine are preferred in that the L-cysteine is too unstable in solution, is endowed with an unpleasant smell and with a fleeting action due to its too quickly metabolization.

Among the most used derivatives of L-cysteine, have to be mentioned according to their decreasing activity "in vitro" the following ones: N-guanylcysteine, N-carbamoylcysteine, Acetylcysteine, Mecysteine and the ethyl ester of the cysteine.

Acetylcysteine is the most used in therapy.

A distinctive feature common to all these sulfurated compounds and essential element for the purpose of the mucolytic activity is the presence of —SH group: in fact, the protection of this group by acetylation or its oxidation to disulfide involve the disappearance of the mucolytic activity (Scheffner A.L.: The reduction "in vitro" in viscosity of mucoprotein solutions by a new mucolytic agent, N-acetyl-L-cysteine. Ann. N.Y. Acad. Sci., *106*, 298, (1963)).

The way of acting of the L-cysteine derivatives, either "in vitro" or by local application by instillation or aerosoltherapy, is ascribed to a thiole-disulfide interaction with opening of the disulfocystinic intramolecular bridges of the mucoproteins and consequent reduction of the viscosity of the mucoproteins themselves. On the contrary, by oral and parenteral administration the action of these drugs seems to be ascribed to a regulating effect on the cellular metabolism and in particular on the enzymatic activities, on the oxido-reductive processes of the respiratory chain and on the membrane permeability of the bronchia where the thiolic compounds tend to concentrate. Also the Carbocysteine, wherein the thiol group is blocked, is endowed with the above mentioned properties, which are mucoregulating properties rather than mucolytic ones.

The compounds of the present invention have unexpectedly proved to be endowed with a tolerability and mucoregulating activity higher than those of Carbocysteine.

More particularly the advantages of the new compounds of the present invention may be summarized as follows:

1) Compounds of formula (I) wherein R is hydrogen: progressive activity together with reduced local irritation due to the absence of free thiol groups and impossibility of giving quickly biodegrading cysteine (essential difference in respect to Carbocysteine).

2) Compounds of formula (I) wherein R is a 2—20 C alkyl radical: high absorption owing to high liposolubility and transmucosal penetrability, high tolerability at the bronchial mucouse as well as at the alveolar epithelium because of the affinity (and particularly in the case of palmitic esters) to the pulmonar surfactant (dipalmitoyl-lecithine) responsible for the preservation of the alveolar physiological characteristics as well as of the pulmonar expansion capacity.

The new derivatives of this invention have proved to be inactive in the test "in vitro" directly based on the viscosimetric variations of the bronchial sputum and/or of the hog gastric mucin.

This result is in accordance with the lack of free thiol groups in the compounds of this invention.

On the contrary, in the tests "in vitro" carried out on the sputum of patients showing a bronchial hypersecretion and treated by oral administration for one week with 30 mg/kg/day of the acids (compounds of formula (I) wherein R = H) or with 50 mg/kg/day of the esters (compounds of formula (I) wherein R = 2—20 C alkyl radical), the new compounds of the invention have proved effective to modify the mucins of the fibrillar reticulum. The analysis of the glycopeptides of the bronchial mucus obtained by hydrolysis with papaine (24 hours at 37°C, pH 6.5) followed by pronasis (24 hours at 37°C at pH 7.3) and fractionation by electrophoresis on agarose at pH 8.6 and finally chromatography on DEAE-Sephadex A/25 column has shown an average increasing of about 100% of the sialoglycopeptides (hydrophilics) with a reduction higher than 60% of the weakly acid glycopeptides and neutral mucins (hydrophobics) followed by lowering of the viscosity. Comparative tests carried out on Carbocysteine at higher doses (70 mg/kg/day) resulted in an average increasing of the sialoglycopeptides not higher than 75% and in a decreasing of the glycopeptides and mucins not higher than 45%. Also in the tests "in vitro" the mucolytic and mucoregulating activity of the new compounds of the invention has proved to be high in the tests in rats for the evaluation of the bronchial hypersecretion induced with $SO_2$: after oral administration for two weeks of 0.5 g/kg/day of the compounds of formula (I) wherein R = H or of an amount of the compounds of the formula (I) wherein R = 2—20 C alkyl radical in an amount equivalent to 0.5 g/kg/day of free acid, the inhalation of 300 ppm of $SO_2$ for two hours a day for a total of 90 hours caused a bronchial obstruction only in the 15% of the treated animals in respect to the 80% of the untreated animals. Under the same experimental conditions in tests carried out with Carbocysteine at the same dose (0.5 g/kg/day) the percentage of the non protected animals was 25%.

By histological and histochemical examinations (P.S.A. coloration) a reduction of about 40% of the globet hyperplasya and of the number of the mucipar cellules together with an about complete persistence of the —SH group (D.D.D. coloration) in the cellules of the bronchial mucousa has been proved in the bronchial mucousa of the rats treated with the new products; this result is to be ascribed to a protective-citothropic action. Finally, in the rats which underwent a $CCl_4$ nebulization for 10 days (5 nebulizations for 2 minutes a day) the contemporaneous treatment by oral route with the compounds of

the formula (I) wherein R = H at the dose of 0.5 g/kg/day or with the compounds of formula (I) wherein R = 2—20 C alkyl radical in an amount equivalent to 0.5 g/kg/day of the acid, hindered for about 60% the intrabronchial desquamation and the reactive mucousa hyperplasye; under the same experimental conditions, the Carbocysteine, at the same dose, resulted in a hindering not higher than 40%. These results are in favour of a protective-trophic action of the new compounds.

All the above reported tests "in vivo" show that the new compounds are endowed with an high mucroregulating action, higher than that of Carbocysteine, with a lowering of the hypersection and of the experimentally induced phlogosis, with riequilibrium of the glycopeptidic fractions of the pathologic mucus, which becomes less viscous after the treatment, but not fluid.

From the toxicological point of view all the new compounds have been proved to have a remarkably low acute toxicity: the S-carboxymethylderivative of the alphathiopropionamido acetic acid buffered with sodium bicarbonate at pH 6 shows a $DL_{50}$ of 5.42 g/kg by i.p. administration in the mouse and the analogous derivative of the beta-thiopropionamido acid shows a $DL_{50}$ of 5.50 g/kg; under the same operative conditions the $DL_{50}$ of the Carbocysteine is 5.18 g/kg that is of the same order size.

The $DL_{50}$ of the aliphatic esters of both the mentioned acids cannot be determined by i.p. route in that it is impossible to administrate the compounds at a dose higher than 5 g/kg; by oral administration in the mouse the $DL_{50}$ is higher than 8 g/kg, that is a value comparable with that of the Carbocysteine.

The subacute, chronic and foetal toxicity have been evaluated by administering the buffered acids (that is the compounds of the formula (I) wherein R = H) dissolved in drinkable water and the esters (compounds of formula (I) wherein R = 2—20 C alkyl radical) mixed with food.

The subacute toxicity tests carried out in rats for 12 weeks, the chronic toxicity tests carried out for 6 months in rats and dogs (0.1—0.9 g/kg/day) as well as the foetal toxicity tests both in female rats (0.3—0.9 g/kg/day from the 5th to 15th days of pregnancy) and in female rabbits (0.1—0.3 g/kg/day from the 6th to 18th day of pregnancy) have proved the excellent tolerability of the new compounds of the present invention.

Inspections on the animals used for the chronic toxicity test did not show any significant variation as to the body weight development, the weight as well as the macro- and microscopic appearance of the main organs, in the haemochromocitomeric tests, in the main tests on the liver functionally (bilirubine, SGOT, SGPT, alkalyphosphatase), in uricemia, chloesteremia, glycaemia, azotemia and standard tests of the urine. Furthermore, the treatments did not negatively affect either mothers or the characteristics of the litters such as for instance the number of borns, the foetal losses and the weight of the litters or of foetus, or the embryofoetal development evaluated through the incidence of malformations, anomalies and skeletal and/or visceral variations.

In order to evaluate the pulmonar tolerability, on the same rats, which previously underwent the tests for chronic toxicity, the influence of the surfactant has been determined at the end of the treatments. By the analysis of the phospholipides qualitative analysis by TLC chromatography and quantitative analysis by determining the content of phosphorus and of the fatty acids (by gas-chromatography) contained in the liposomes separated by centrifugation from the washing liquid of the excised lungs, no significant variation has been observed in respect to the controls.

No significant difference has been noticed, in respect to the controls, as to the amount of pneumocites of the II type, in their content in granules (fixation and coloration with Os-I).

The pharmacocinetic tests (titrations in the urines after oral administration of 100 mkg/kg of acids and 200 mg/kg of esters in rats have shown a preferential renal excretion in unmodified form for at least 25% of the administered dose and for about 25% as organic sulfurated not sulfide compounds.

This behaviour represents a substantial difference in respect to other thiols (derivatives of cysteine, cysteamine, penicillamine) which on the contrary are usually mainly excreted as inorganic sulfates in that those thiols are completely metabolized.

The results show the poor biodegradation of the new compounds of the present invention analogously to Carbocysteine and differently from the thiolic compounds. In the clinical field, preliminary tests with the acids by aerosol (0.5 g) and oral route (2 g) buffered or neutralised with Ca and with the esters by oral (3 g) or rectal route (0.9 g) a day for at least one week in chronical bronchitic patients have shown a progressive lowering of the sputum viscosity (on the average 70% as to the purulent sputum and 50% respectively as to the muco-purulent sputum) at the 4th-5th day of treatment (determinations by means of the flat-cone rotating viscosimeter) with an average increasing of 20% of the vital capacity, of 15% of the $F.E.V_1$ and lowering of the residue volume of about 20%.

The chinine action of the kallikrein on the bronchial tone was not observed with the alpha- and beta-acids buffered or neutralized with Ca and administered by aerosol; in other words, no bronchospasm has been registered (determination by the plethysmograph in patients suffering from broncholability). This test also proved the excellent topic tolerability of the new compounds of the invention.

The results obtained with the acids neutralized with Ca or buffered with sodium hydroxide are comparable: however the former are characterized by a more remarkable antiphlogistic effect because of the known action of the calcium ion. Furthermore, the acids neutralized with Ca are more suitable for the rectal administration especially as to the local tolerability in formulations with hydrophilic vehicles.

Still more suitable for the rectal use are the esters of fatty alcohols, in particular the esters of lauryl, myristyl and cetyl alcohol.

These esters, when formulated as suppositories containing triglycerids resulted very well tolerated and as active as the corresponding alpha- and beta-thiopropionamidoacetic-S-carboxymethylated acids.

The above esters may be administered also by aerosol, dispersed in a 5—10% solution of 0.15 M sodium chloride; they are transformed into aerosol by a supersonic waves apparatus at 37°C with formation of submicronic particles (0.1—0.6 micron) endowed with high stability able to ensure the penetration of other drugs, such as chemio-antibiotics, into the pulmonar alveolus.

In order to illustrate the present invention we will give hereinafter some examples of typical formulations based on the compounds according to the present invention.

Example 3

Vials or ampoules for aerosol

| | |
|---|---|
| S-carboxymethylderivate of the alpha- or beta-thiopropionamidoacetic acid buffered with NaOH or $Na_2CO_3$ or neutralized by Ca | 250 mg |
| sterilized distilled water q.s. to | 3 ml |

Average Dosage: 2 vials a day

Example 4

Tablets

| | |
|---|---|
| (a) S-carboxymethylderivate of the alpha- or beta-thiopropionamido acetic acid buffered with NaOH or $Na_2CO_3$, or neutralized by Ca | 500 mg |
| starch and lactose q.s. to | 0.8 g |
| (b) S-carboxymethylderivate of the alpha- or beta-thiopropionamidoacetic acid esterified with lauryl or myristyl or cetyl alcohol | 750 mg |
| ethylcellulose | 150 mg |

Average Dosage: 4 tablets a day

Example 5

Syrup

| | |
|---|---|
| S-carboxymethylderivate of the alpha- or beta-thiopropionamidoacetic acid buffered with NaOH or $Na_2CO_3$, or neutralized with Ca | 5 g |
| methyl p-hydroxybenzoate | 0.15 g |
| flavoured solution q.s. to | 100 ml |

Average Dosage: adults: 3 spoon a day; children: half dose

Example 6

Suppositories

| | |
|---|---|
| (a) S-carboxymethylderivate of the alpha- or beta-thiopropionamidoacetic acid buffered with NaOH or $Na_2CO_3$, or neutralized with Ca | 300 mg |
| polyethylenglycol q.s. to | 3 g |

(b) S-carboxymethylderivate of the alpha- or beta-thiopropionamidoacetic acid esterified with lauryl or myristyl or cetyl alcohol 300 mg

triglycerides of fatty acids q.s. to 3 g

Average Dosage: adults: 3 a day; children: half dose.

Furthermore, the compounds of formula (I)

$$R_2OOC—CH_2—S— CH —C—NH—CH_2—COOR \qquad (I)$$

$$R_1 \; n \; O$$

wherein R, $R_1$, $R_2$ and *n* have the above defined meanings, act on the skin showing a high antiseborrheoic action which favours the cleansing and hygiene of the fatty skin affected with boils, comedones and acne; furthermore they show an antidandruff action and favour the regeneration of the weak and trichoclasic hair.

The antiseborrheoic action of the compunds represented by the formula (I) has been tested by oral administration of the above mentioned compounds to rats under hyperseborrheoic conditions. The tests have been carried out by administration to the rats under hyperseborrheoic conditions because of a diet poor in biotin (rich in white of egg), doses comprised between 0.1 and 0.9 g/kg/day of the compounds according to the invention.

The compounds of formula (I) wherein $R=R_2=H$ have been tested by administration with drinkable water buffered with sodium carbonate; the compounds wherein $R_2$ is H or $\frac{1}{2}$ Ca and R is a lauryl, myristyl or cetyl radical, have been tested by administering them mixed with food.

Comparable results were obtained in all the cases: 15 days after the treatment the seborrheoic lesions appeared reduced as to intensity and wideness; furthermore the fur appeared dried and stronger neither sticky, nor opaque, nor bristly; the skin appeared brighter and unwinkled in comparison with the skin of the untreated animals.

Under autoptic examination the liver of the treated animals appeared normal, whereas the liver of the untreated animals appeared hypertrophic with degenerative alterations typical of the lacking of biotin. The histological examinations of the skin of the treated animals showed a clear reduction of the volume of the sebaceous glands which in the untreated animals appeared bulky and frequently broken.

The determination of the skin lipides has shown an average lowering of about 20% of the skin lipides of the treated animals in comparison with the skin lipides of the untreated ones.

Furthermore, the capability to reduce the seborrheoic hypersecretion of the skin has been investigated as to the compound of the formula (I) by topical application on the skin of suitable formulations, upon testing of the local tolerability, acute and subchronic local toxicity as well as of the systemic toxicity. In order to estimate the possible local irritating effect a 12 × 7 cm area of the skin of the abdomen of albino rabbits (average weight 1.500 kg) has been clipped.

The fore end of the clipped area has been abraded by means of abrasive paper (about 2 $cm^2$), without causing bleedings. On the abraded zone and on a zone of unabraded skin at the lack end of the clipped area of each rabbit, an amount of about 0.5 g for single composition according to the formulations from 7 to 12 has been spread as homogeneous layer. 24 hours after the application the composition has been removed and the skin below has been tested both immediately and after 24 and 48 hours respectively to estimate the skin irritation scores caused by the applied compositions. The skin of all the treated animals appeared wholly normal, without apparent differences between the unabraded and the abraded zone.

In order to evaluate the acute skin toxicity, on the previously clipped abdomen of albino rabbits (15 × 5 cm) the skin has been abraded on two lines at the sides of the median line and in number of 5 for each line. The skin lesions were enough deep to affect the corneous layer, but did not bleed.

On the clipped area of each animal (about 105 $cm^2$) the compositions of the formulations from 7 to 12 have been spread in an amount of about 5 g for single animal. The treated skin was covered with a gauze secured in position by wrapping with a band. The evaluation of the skin condition, carried out after a 24 hours period of exposure to the above compositions, did not show in any case clear lesion scores.

In order to evaluate the subchronic skin toxicity, the abdomen skin of albino rabbits has been clipped (about 12 × 7 cm) 0.5 g of the compositions of the formulations from 7 to 12 has been spread each day on the clipped areas for 25 days. The area has been exposed in air. The observation of the skin, carried out at the end of the treatment period, did not show any local irritation score. In order to evaluate possible systemic effects following from the subchronic treatment, the rectal temperature of the treated animals has been measured each seven day.

The observed variations keeped always within ± 1.5°C compared with the starting temperature;

the rectal temperature of the untreated rabbits kept under the same stalling conditions remained within the same limits. At the end of the treatment period the urines of the treated rabbits have been separately collected and used to determine the albumin, glucose, ketones, bile pigments and occult blood. All these tests gave no variation in comparison with the untreated animals. The weight variations of the animals during the observation period were not remarkably different from the weight variations of the untreated animals.

In order to evaluate the capability of the new compounds according to the present invention to reduce the seborrheoic skin hypersecretion, hyperseborrheoic conditions have been induced in albino rabbits by a diet poor in biotin.

The abdomen skin (about 12 × 7 cm) of the rabbits have been clipped. Half the clipped area has been spread with about 0.5 g of each of the compositions described in the examples from 7 to 12, whereas the other half of the clipped area has been spread only with the vehicles for the sake of comparison.

The application has been repeated every day during 25 days. After this period of time it has been observed that on the hemiabdomen treated only with the vehicles of the compositions referred to in formulations from 7 to 12 the fur was much more opaque, bristly and sticky and the skin more flaccid and wrinkled than on the hemiabdomen treated with the compositions containing the active ingredient according to the present invention. From the histological examination of the skin treated with the new compounds according to the invention appeared that, differently from what has been observed on the skin treated only with the vehicles, the connective tissues is in good state with well interwisted bundles; no pathological infiltrating elements are observed; the muscular system appears undamaged. Some examples of formulations are given hereinafter; these formulations have been tested in human field and they in all cases have shown a clear antiseborrheoic action; soaps, gels and creams favour the cleansing and hygiene of the fatty skins and therefore hinders the appearance of boils, comedones as well as acne. Shampoos and lotions have shown in all cases a clear antidandruff action coupled with an action which favours the structuration of the weakened hair as well as of trichoclasic hair.

Obviously the formulations referred to hereinafter are merely given for sake of illustration in that many other formulations can be thought within the scope of the present invention.

The final pH of all the compositions is 5.5 ± 0.3 and they are added with a perfume or an hypoallergic aromatizing compound as well as with preservatives such as alkyl-p-hydroxybenzoates, benzyl alcohol etc. The preservatives are not requested for the formulations of antiseptic creams and antiseptic lotions if these formulations already contain antibacterial agents.

Example 7

Cream soap

| | |
|---|---|
| S-carboxymethylderivative of the alpha-thiopropionamidoacetic acid buffered with NaOH or $Na_2CO_3$ | 0.4 g |
| Lecithin | 0.7 g |
| Dodecyl-ethylen-glycol monolaurate | 5.0 g |
| Hamamelis water | 18.0 g |
| Camomile water | 18.0 g |
| Distilled water q.s. ad 100 g | |

This soap is suitable for the cleansing of fatty skin with boils, comedones as well as for the hygiene and treatment of fatty skins with disposition to acne.

Soap showing equivalent action can be prepared also by replacing the beta-thiopropionamidoacetic acid by the alpha-thiopropionamidoacetic acid.

Calcium salts of these acids can be also used.

Example 8

Liquid soap

| | |
|---|---|
| S-carboxymethylderivative of the alpha-thiopropionamidoacetic acid buffered with NaOH or $Na_2CO_3$ | 0.5 g |
| Acetyl-dimethyl-dodecylammonium chloride | 28.0 g |
| Distilled water q.s. ad 100 g | |

Also this soap is suitable for cleansing fatty skins with boils and comedones.

Equivalent soaps can be prepared by using the beta-thiopropionamidoacetic acid as such or in the form of its calcium salt or the calcium salt of the alpha-thiopropionamidoacetic acid.

Example 9

Gel

| | |
|---|---|
| S-carboxymethylderivative of the alpha-thiopropionamidoacetic acid buffered with NaOH or $Na_2CO_3$ | 1.5 g |
| Carboxypolymethylene | 1.5 g |
| Triethanolamine | 1.5 g |
| Water q.s. ad 100 g | |

The sodium salt may be replaced by the Ca salt.

The salt of the S-carboxymethylderivative of the beta-thiopropionamidoacetic acid may be also used with the same results.

Also this information is advantageously used for the treatment of fatty skins with disposition to acne.

Example 10

Gel

| | |
|---|---|
| S-carboxymethylderivative of the alpha-thiopropionamidoacetic acid esterified with lauryl alcohol | 2.5 g |
| Carboxypolymethylene | 3.0 g |
| Triethanolamine | 3.5 g |
| Isopropyl alcohol | 30.0 g |
| Polyoxyethylen(20)-sorbitan-monolaurate | 5.0 g |
| Distilled water q.s. ad 100 g | |

Myristyl or cetyl esters of the alpha-thiopropionamidoacetic acid may be also used as well as the lauryl, myristyl or cetyl esters of the beta-thiopropionamidoacetic acid.

Example 11

Hydrophilic ointment

| | |
|---|---|
| S-carboxymethylderivative of the alpha-thiopropionamidoacetic acid esterified with lauryl alcohol | 2.5 g |
| Polyglycol 4000 | 11.2 g |
| Stearyl alcohol | 20.8 g |
| Glycerine | 17.1 g |
| Sodium laurylsulphate | 0.6 g |
| Distilled water q.s. ad 100 g | |

The myristyl or cetyl ester of the alpha-thiopropionamidoacetic acid can be also used as well as the corresponding esters of the beta-thiopropionamidoacetic acid.

Ointments according to the above formulation have proved to be very useful for the treatment of dry skins with disposition to acne.

Example 12

Antiseptic cream (emulsified lotion)

| | |
|---|---|
| S-carboxymethylderivative of the alpha-thiopropionamidoacetic acid buffered with NaOH or $Na_2CO_3$ | 1.5 g |
| Cetyl-trimethylammonium bromide | 0.2 g |
| Cetyl alcohol | 1.5 g |
| White bees wax | 0.1 g |
| Glycerine | 5.0 g |
| Sodium laurylsulphate | 0.5 g |
| Distilled water q.s. ad 100 g | |

The active sodium salt may be replaced by an equivalent amount of the calcium salt or by the sodium or calcium salt of the corresponding beta-thiopropionamidoacetic acid derivative.

The compositions are particularly useful for the disinfection of fatty skins with boils and comedones, as well as for the hygiene and treatment of fatty skin with disposition to acne.

Example 13

Antiseptic lotion

| | |
|---|---|
| S-carboxymethylderivative of the alpha-thiopropionamidoacetic acid buffered with NaOH or $Na_2CO_3$ | 3.0 g |
| Cetyltrimethylammonium bromide | 0.1 g |
| Polyoxymethylen(20)-sorbitan-monolaurate | 8.5 g |
| Ethyl alcohol 96° and water q.s. ad 100 g to obtain an alcoholic grade of | 12.5° |

Compositions obtained with the calcium salt or with salts of the corresponding beta-thiopropionamidoacetic acid derivative have been proved to be all equally useful for the disinfection of the fatty skins with boils and comedones and for the hygiene and treatment of fatty skins with disposition to acne.

Example 14

Cream shampoo (for dry hair and trichoclasis)

| | |
|---|---|
| S-carboxymethylderivative of the alpha-thiopropionamidoacetic acid esterified with lauryl alcohol | 5.0 g |
| Magnesium stearate | 1.0 g |
| Sodium laurylsulphonate | 30.0 g |
| Polyvinylalcohol (10%) | 20.5 g |
| Methylcellulose | 9.0 g |
| Glycerylmonolaurate | 1.0 g |
| Lanolin | 0.5 g |
| Distilled water q.s. ad 100 g | |

Equally effective compositions have been obtained by using the myristyl or cetyl ester, or the esters of the beta-thiopropionamidoacetic acid derivative.

## Example 15

Liquid shampoo (for fatty hair)

| | |
|---|---|
| S-carboxymethylderivative of the alpha-thiopropionamidoacetic acid buffered with NaOH or $Na_2CO_3$ | 5.0 g |
| Triethanolamine laurylsulfate | 35.0 g |
| Sodium alginate | 2.5 g |
| Distilled water q.s. ad 100 g | |

Equally effective liquid shampoos have been obtained by using the calcium salt or the corresponding salts of the S-carboxymethylderivative of the beta-thiopropionamidoacetic acid.

## Example 16

Liquid shampoo (for fatty hair)

| | |
|---|---|
| S-carboxymethylderivative of the alpha-thiopropionamidoacetic acid buffered with NaOH or $Na_2CO_3$ | 5.0 g |
| Coconut-oil | 14.0 g |
| Olive oil | 3.0 g |
| Castor oil | 3.0 g |
| Potassium hydroxide (85%) | 4.7 g |
| Glycerine | 2.0 g |
| Ethyl alcohol (96°) | 4.0 g |
| Sodium hexametaphosphate | 1.0 g |
| Distilled water q.s. ad 100 g | |

Equally effective shampoos have been obtained by using the corresponding calcium salt or the corresponding salts of the S-carboxymethylderivative of the beta-thiopropionamidoacetic acid.

## Example 17

Hair tonic lotion

| | |
|---|---|
| S-carboxymethylderivative of the alpha-thiopropionamidoacetic acid buffered with NaOH or $Na_2CO_3$ | 5.0 g |
| Polyoxyethylen(20)-sorbitan-monolaurate | 6.0 g |
| Ethyl alcohol (96°) and water q.s. ad 100 g | |

Equivalent formulations have been prepared by using the corresponding myristyl or cetyl esters, or the lauryl, myristyl or cetyl ester of the S-carboxymethylderivative of the beta-thiopropionamidoacetic acid.

## Claims

1. Derivatives of the mercaptopropionamido-acetic acid of the formula

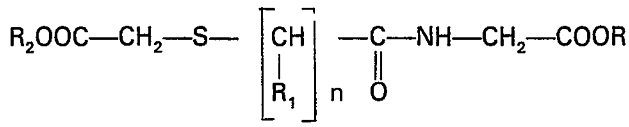

(I)

wherein R is H or 2—20 C alkyl group; $R_1$ is H or $CH_3$, *n* is 1 or 2, with the provision that when n = 1, $R_1$ is $CH_3$ and when n = 2, $R_1$ is H; $R_2$ is H or $\frac{1}{2}$ Ca.

2. Derivatives of the alpha-mercaptopropionamido-acetic acid of the formula

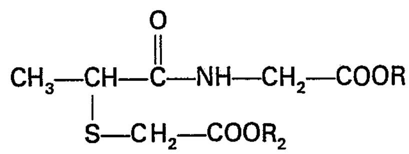

(II)

wherein R is H or 2—20 C alkyl group; $R_2$ is H or $\frac{1}{2}$ Ca.

3. Derivatives of the beta-mercaptopropionamido-acetic acid of the formula

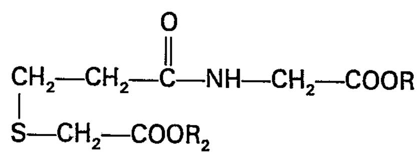

(III)

wherein R is H or 2—20 C alkyl group; $R_2$ is H or $\frac{1}{2}$ Ca.

4. A process for the preparation of derivatives of the mercaptopropionamido-acetic acid of the formula

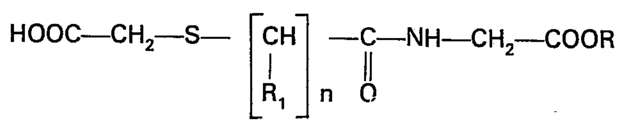

wherein R is H or 2—20 C alkyl group; $R_1$ is H, $CH_3$; *n* = 1 or 2 with the provision that when n = 1, $R_1$ = $CH_3$ and when n = 2, $R_1$ = H, characterized in that: (a) glycine or an ester thereof is reacted with a halide of a bromopropionic acid according to the reaction scheme:

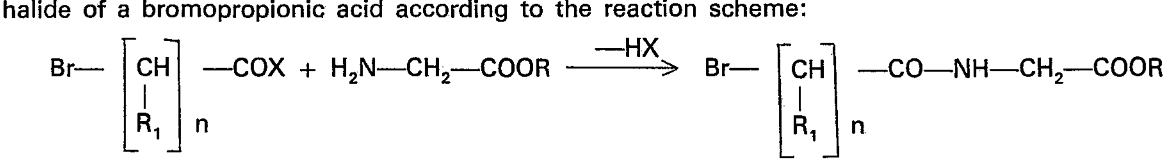


(wherein R, $R_1$ and *n* have the above meanings; X = Cl, Br, I)
and (b) the thus obtained derivative of the bromo-propionamido-acetic acid is reacted with thioglycolic acid according to the scheme:

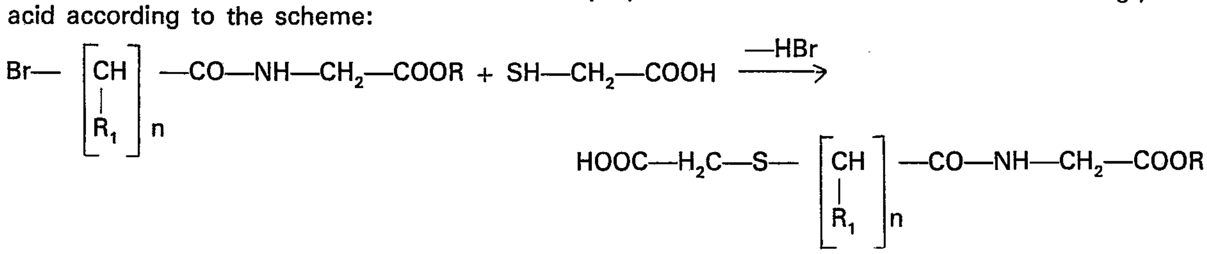


5. A process according to claim 4, characterized in that the step (a) of the process carried out in an inert organic solvent, immiscible with water, in the presence of an organic base in at least stoichiometric amount on the starting materials, at a temperature between 0° and 10°C.

6. A process according to claim 4, characterized in that the step (b) of the process is carried out in an aprotic dipolar organic solvent, water-miscible, in the presence of an organic or inorganic base in at least stoichiometric amount on the thioglycolic acid, at room temperature.

7. Therapeutical mucolytic compositions, characterized in that the compositions contain a compound of the general formula

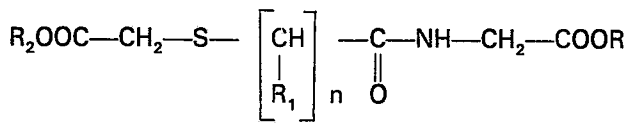

(wherein R is H or 2—20 C alkyl group; $R_1$ is H, $CH_3$; $n = 1$ or 2 with the provision that when n = 1, $R_1 = CH_3$ and when n = 2, $R_1 = H$; $R_2 = H$ or $\frac{1}{2}$ Ca) as active ingredient together with pharmaceutically acceptable vehicles and diluents.

8. Antiseborrheoic and antidandruff compositions, characterized in that they contain a compound of the general formula

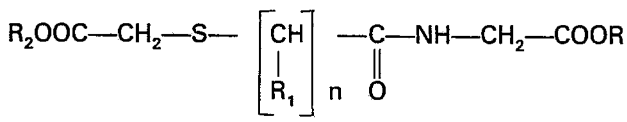

(wherein R is H or 2—20 C alkyl group; $R_1$ is H or $CH_3$, $n = 1$ or 2 with the provision that when n = 1, $R_1 = CH_3$ and when n = 2, $R_1 = H$; $R_2 = H$ or $\frac{1}{2}$ Ca) as active ingredient together with suitable vehicles and diluents.

**Patentansprüche**

1. Derivate der Merkaptopropionamidoessigsäure der Formel

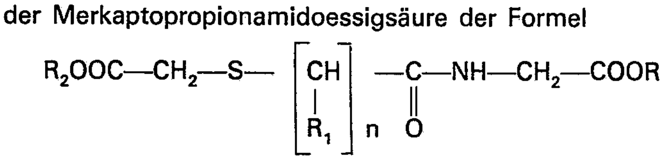 (I)

worin R = H oder ein 2 bis 20 C enthaltender Alkylrest bedeutet; für $R_1$ H oder $CH_3$ steht, $n = 1$ oder 2, unter der Bedingung, dass, wenn $n = 1$, $R_1$ $CH_3$ ist und wenn n=2, $R_1$ H ist; $R_2$ H oder 1/2 Ca bedeutet.

2. Derivate der Alpha-Merkaptopropionamidoessigsäure der Formel

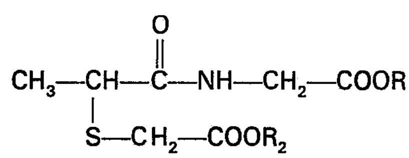

worin R H oder ein 2 bis 20 C enthaltender Alkylrest bedeutet; für $R_2$ H oder 1/2 Ca bedeutet.

3. Derivate der Beta-Merkaptopropionamidoessigsäure der Formel

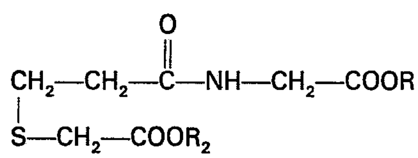

worin R H oder ein 2 bis 20 C enthaltender Alkylrest bedeutet; für $R_2$ H oder 1/2 Ca bedeutet.

4. Verfahren zur Herstellung von Derivaten der Merkaptopropionamidoessigsäure der Formel

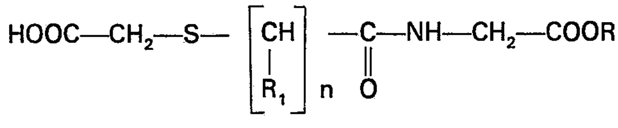

worin R H oder ein 2 bis 20 C enthaltender Alkylrest bedeutet; für $R_1$ H, $CH_3$ steht; $n = 1$ oder 2, unter der Bedingung, dass, wenn n=1, $R_1=CH_3$, und wenn n=2, $R_1$ H ist, dadurch gekennzeichnet, dass: (a) entweder Glyzin oder ein Ester desselben mit einem Halogenid einer Brompropionsäure gemäss folgendem Reaktionsschema reagiert:

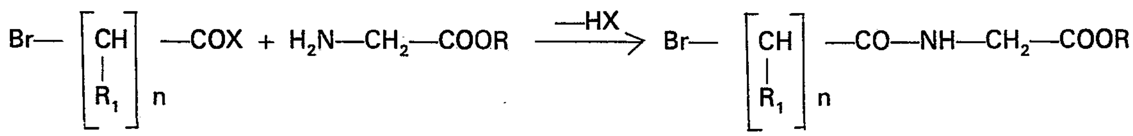


(worin R, $R_1$ und $n$ die oben erwähnten Bedeutungen haben; X = Cl, Br, I)
und (b) das so erhaltene Derivat der Brompropionamidoessigsäure mit Thioglykolsäure gemäss folgendem Reaktionsschema:

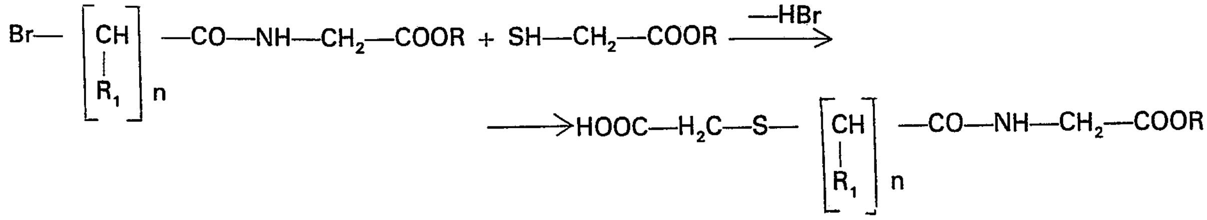

reagiert.

5. Verfahren nach Anspruch 4, dadurch gekennzeichnet, dass Verfahrensschritt (a) in einem mit Wasser nicht mischbaren, inerten, organischen Lösungsmittel, in Anwesenheit einer organischen Base in einer mindestens stöchiometrischen Menge bezogen auf die Ausgangsprodukte, bei einer Temperatur zwischen 0° und 10°C, durchgeführt wird.

6. Verfahren nach Anspruch 4, dadurch gekennzeichnet, dass Verfahrensschritt (b) in einem wassermischbaren, aprotischen, dipolaren, organischen Lösungsmittel, in Anwesenheit einer organischen oder anorganischen Base in einer mindestens stöchiometrischen Menge bezogen auf die Thioglykolsäure, bei Raumtemperatur, durchgeführt wird.

7. Therapeutische mukolytische Arzneimittel, dadurch gekennzeichnet, dass sie eine Verbindung der allgemeinen Formel

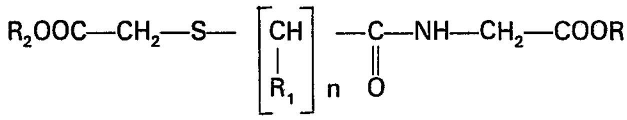

(worin R H oder ein 2 bis 20 C enthaltender Alkylrest bedeutet; $R_1$ H oder $CH_3$ bedeutet; $n$ = 1 oder 2 unter der Bedingung, dass, wenn n=1, $R_1$=$CH_3$ und wenn n=2, $R_1$=H; $R_2$ H oder 1/2 Ca bedeutet) als Wirkstoff zusammen mit pharmazeutisch verträglichen Trägern und Verdünnungsmittel enthalten.

8. Seborrhöe- und Schuppenbekämpfende Arzneimittel, dadurch gekennzeichnet, dass sie eine Verbindung der allgemeinen Formel

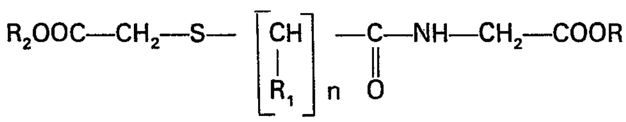

(worin R H oder ein 2 bis 20 C enthaltender Alkylrest bedeutet; $R_1$ H oder $CH_3$ ist; $n$ = 1 oder 2 unter der Bedingung, dass, wenn n=1, $R_1$ = $CH_3$ und wenn n=2, $R_1$=H; $R_2$ H oder 1/2 Ca bedeutet) als Wirkstoff zusammen mit geeigneten Trägern und Verdünnungsmitteln enthalten.

**Revendications**

1. Dérivés de l'acide mercaptopropionamido-acétique de formule:

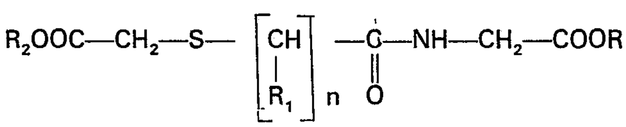

(I)

dans laquelle R est un atome d'hydrogène ou un groupement alkyle en $C_2$—$C_{20}$; $R_1$ est un atome d'hydrogène ou $CH_3$; $n$ est 1 ou 2, sous réserve que lorsque n=1, $R_1$ est $CH_3$ et lorsque n=2, $R_1$ est H; $R_2$ est un atome d'hydrogène ou 1/2 Ca.

2. Dérivés de l'acide alpha-mercaptopropionamido-acétique de formule:

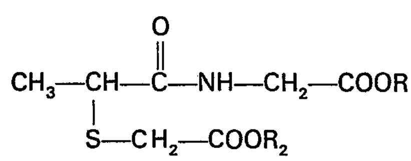

dans laquelle R est un atome d'hydrogène ou un groupement alkyl en $C_2$—$C_{20}$; $R_2$ est un atome d'hydrogène ou 1/2 Ca.

3. Dérivés de l'acide beta-mercaptopropionamido-acétique de formule:

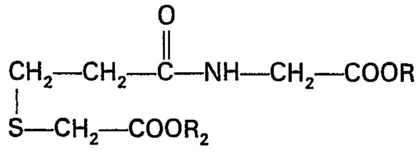

dans laquelle R est un atome d'hydrogène ou un groupement alkyle en $C_2$—$C_{20}$; $R_2$ est un atome d'hydrogène ou 1/2 Ca.

4. Procédé de préparation des dérivés de l'acide mercaptopropionamido-acétique de formule:

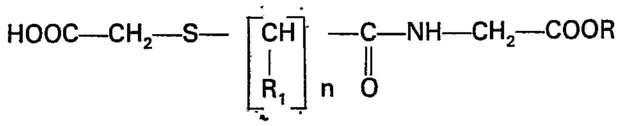

dans laquelle R est un atome d'hydrogène ou un groupement alkyle en $C_2$—$C_{20}$; $R_1$ est un atome d'hydrogène ou $CH_3$; *n* est 1 ou 2, sous réserve que lorsque n=1, $R_1$ est $CH_3$ et lorsque n=2, $R_1$ est un atome d'hydrogène, caractérisé en ce qu'il consiste (a) à faire réagir de la glycine ou un ester de la même avec un halogénure d'un acide bromopropionique selon la schéma de réaction suivant:

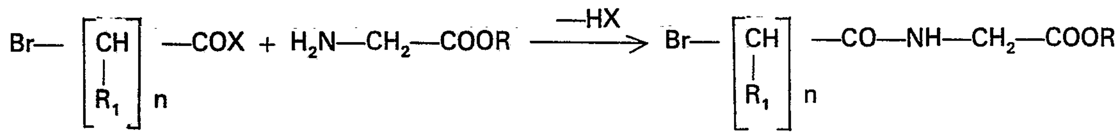


(dans lequel R, $R_1$ et n sont tels que définis plus haut; X est Cl, Br, I)
et (b) à faire réagir le dérivé de l'acide bromo-propionamido acétique ainsi obtenu avec l'acide thioglycolique selon le schéma suivant:

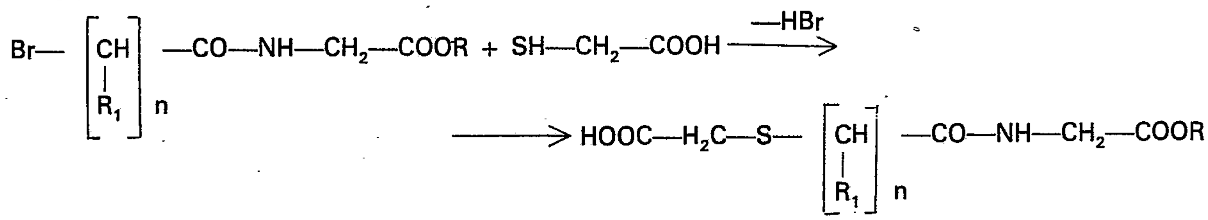


5. Procédé selon la revendication 4, caractérisé en ce que la phase (a) du procédé est réalisée dans un solvant organique inerte, non-miscible avec l'eau, en présence d'une base organique en quantité au moins stoechiométrique calculée sur les matières premières, à température entre 0° et 10°C.

6. Procédé selon la revendication 4, caractérisé en ce que la phase (b) du procédé est réalisée dans un solvant organique dipolaire aprotique, miscible avec l'eau, en présence d'une base organique ou inorganique, en quantité au moins stoechiométrique calculée sur l'acide thioglycolique, à température ambiante.

7. Compositions thérapeutiques, mucolytiques, caractérisées en ce qu'elles contiennent un composé de formule générale:

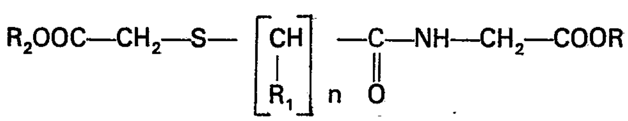

(dans laquelle R est un atome d'hydrogène ou un groupement alkyle en $C_2$—$C_{20}$; $R_1$ est un atome d'hydrogène ou $CH_3$; *n* est 1 ou 2, sous réserve que lorsque n=1, $R_1$ est $CH_3$ et lorsque n=2, $R_1$ est hydrogène; $R_2$ est un atome d'hydrogène ou 1/2 Ca) en tant qu'élément actif avec des excipients pharmaceutiquement acceptables et des diluants.

8. Compositions antiséborrhéiques et antipellicules, caractérisées en ce qu'elles contiennent un composé de formule générale:

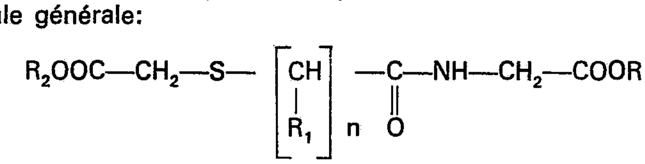

(dans laquelle R est hydrogène ou un groupement alkyle en $C_2$—$C_{20}$; $R_1$ est un atome d'hydrogène ou $CH_2$, *n* est 1 ou 2 sous réserve que lorsque n=1, $R_1$=$CH_3$ et lorsque n=2, $R_1$ est un atome d'hydrogène; $R_2$ est un atome d'hydrogène ou 1/2 Ca) en tant qu'élément actif avec des appropriés excipients et diluants.